# EUROPEAN PATENT APPLICATION

(11) **EP 4 618 589 A1**
(43) Date of publication of application: **17.09.2025**
(21) Application number: 24163260.3
(22) Date of filing: 13.03.2024
(51) Int. Cl.: H04R 1/10, H04R 1/28

(54) **COMPACT ACOUSTIC DEVICE WITH ADAPTABLE FREQUENCY CONTROL**

(71) Applicant: Sonova AG, 8712 Stäfa (CH)
(72) Inventor: LODDER, Joost, 8712 Staefa (CH); HOFSTEDE, Angelique, 8712 Staefa (CH)
(74) Representative: V.O.

(57) **Abstract**

An acoustic device (100) and method of using such device. A sound channel (11) extends through a housing (10) of the acoustic device (100) between a sound inlet (11i) and a sound outlet (11o). The sound inlet (11i) is configured to receive sound input (Si) from an external environment (Ex). The sound outlet (11o) is configured to deliver sound output (So) that has passed through the sound channel (11) to an ear canal (Ec). A resonance cavity (C) is enclosed within the housing (10) and in acoustic communication with the sound channel (11) for causing an acoustic resonance (A) in a frequency transmission spectrum (So/Si) of sound passing through the sound channel (11). An adjustment mechanism (12) is configured to adjust a size (L1,L2) of the resonance cavity (C) for controlling a frequency (F) of the acoustic resonance (A) in the frequency transmission spectrum (So/Si).

## Description

### TECHNICAL FIELD AND BACKGROUND

The present disclosure relates to acoustic devices and methods of using such devices, e.g. to control a frequency transmission spectrum of sound passing through the acoustic device by means of an adjustable resonance cavity or chamber.

As background, WO 2018/117821 A1 describes an earplug and method for attenuating sound. An acoustic canal extends straight through a housing to guide the sound inside an ear canal. The acoustic canal comprises a canal section dimensioned to fit at least partially inside the ear canal. The canal section ends at one side in a first exterior opening to let the sound into the ear canal. Another side of the canal section transitions into a first resonance volume with a relatively wide diameter. A second resonance volume ends at one side in a second exterior opening and, the other side being separated from the first resonance volume by a sound attenuating mesh. This provides resonance cavities that compensate attenuation of the mesh over a wide range of high frequencies. In one embodiment, two parts of the earplug fit together with a controllable distance defining a variable volume of the first resonance cavity. In this way, the resonance frequency may be tuned. For example, the parts may fit together with a bayonet or screw connection wherein a turning of one part with respect to the other determines the resonance volume and frequency. Alternatively, a simple sliding connection can be provided, e.g. wherein the parts fit tightly together. By allowing user-control over the volume of the resonance cavity, the corresponding resonance frequency can be adapted to a specific ear shape or preference of the user.

As further background, US 10,821,027 B2 relates to devices for filtering sound, and in particular to wearable devices for notch filtering sound. The sound-filtering device comprises an entry port configured to be exposed to an environment to receive sound from the environment when the device is coupled to a portion of an ear of a user; an exit port configured to deliver sound that has passed through the device to an ear canal of the user when the device is coupled to the portion of the ear of the user; a channel extending between the entry port and the exit port to define a path along which sound travels from the entry port to the exit port; a side port in fluid communication with the channel; and a resonant chamber offset from the path defined by the channel and in fluid communication with the channel via the side port. The resonant chamber is configured to attenuate at least a portion of any sound passing through the channel that is within a band of frequencies narrower than a full audible spectrum of the user. A geometric volume of the resonant chamber is selectively adjustable. The resonant chamber is disposed outside the ear canal of the user when the device is coupled to the portion of the ear of the user.

The present disclosure aims to alleviate disadvantages of the prior art, while maintaining at least some of the advantages. For example, it is desired to provide a compact acoustic device offering adaptable control over the frequency transmission spectrum.

### SUMMARY

Aspects of the present disclosure relate to an acoustic device and method of using such device. The acoustic device comprises a housing with a sound channel extending through the housing between a sound inlet and a sound outlet. The sound inlet is configured to receive sound input from an external environment. The sound outlet is configured to deliver sound output that has passed through the sound channel to an ear canal. The acoustic device comprises a resonance cavity enclosed in the housing. The resonance cavity is in acoustic communication with the sound channel. This may cause an acoustic resonance in a frequency transmission spectrum of sound passing through the sound channel. An adjustment mechanism is configured to adjust a size of the resonance cavity. This may be used for controlling a frequency of the acoustic resonance in the frequency transmission spectrum.

By enclosing the adjustable resonance cavity within the housing, the size of the resonance cavity may be varied without changing a size of the housing. For example, the adjustable resonance cavity may be fully enclosed within a fixed outer hull of the housing. Accordingly, an overall size or outer dimension of the acoustic device and/or housing may be maintained while an inner dimension and/or volume of the resonance cavity is varied. For example, instead of using an adjustable piston sticking out of the housing, the adjustment mechanism may be used to control an inner dimension and/or volume of the resonance cavity without changing the overall size of the acoustic device. Preferably, the cavity is formed as part of the sound channel. By arranging the resonance cavity in line with the sound channel, enclosed in the housing, the resonance cavity may be used to generate an acoustic resonance peak, e.g. enhancing sound at an adjustable frequency. Also other or further ways of having the cavity in acoustic communication with the sound channel can be envisaged. By arranging the resonance cavity as a side branch of the sound channel, enclosed in the housing, the resonance cavity may be used to generate an acoustic resonance dip, e.g. attenuating sound at an adjustable frequency.

By providing an adjustment mechanism configured to adjust the size of the resonance cavity in a continuous range of values between a maximum size and minimum size, a position of the acoustic resonance may be set to any desirable value corresponding to the continuous range. By providing the resonance cavity with an adjustable length, the size of the resonance cavity can be easily set using a respective adjustment mechanism. By making the adjustment mechanism externally accessible from outside the housing the size of the resonance cavity, may be manually adjusted. Accordingly, the device does not require any battery powered. Alternatively, an electrical adjustment mechanism may be used. By providing the adjustment mechanism with a knob or slider the size of the resonance cavity may be easily adjusted. By providing a one or more indications of a respective sound setting, a user may control the device according to the indicated setting. For example, a scale may be indicated on the housing, e.g. wherein a knob or slider can be controllable moved to a specific setting.

By forming the resonance cavity as a tubular chamber extending along a circular trajectory, the housing can remain relatively compact while allowing a relatively long resonance cavity. Accordingly, the acoustic resonance may be tuned to lower frequencies of interest. A length of the tubular chamber along the circular trajectory may be adjustable by the adjustment mechanism for controlling the frequency of the acoustic resonance in the frequency transmission spectrum. For example, the adjustment mechanism may comprise and/or or couples to a rotatable part of the acoustic device. By making the adjustment mechanism externally accessible from outside the housing the size of the resonance cavity, may be manually adjusted. Accordingly, the device does not require any battery powered. Alternatively, an electrical adjustment mechanism may be used. By providing the adjustment mechanism with a knob or slider the size of the resonance cavity may be easily adjusted.

By forming the housing of different parts, these parts may be moveable with respect to each other for changing the size of the resonance cavity. Preferably, the housing comprises a rotatable part and a static part. Accordingly, the adjustment mechanism may effect rotation of the rotatable part with respect to the static part so that the rotation may change the size of the resonance cavity. For example, the rotatable part is configured to rotate with respect to the static part along a circular trajectory, wherein the circular trajectory coincides with a part of the sound channel forming the resonance cavity. By rotating the first channel block inside the sound channel along the circular trajectory when the first part of the housing is rotated with respect to the second part of the housing, and keeping the second channel block static inside the sound channel when the first part of the housing is rotated with respect to the second part of the housing, a variable length of the resonance cavity may be determined between a first channel block and a second channel block. By placing the respective channel blocks adjacent to the sound inlet and outlet, respectively, further branching of the channel into a dead space may be prevented.

By fixedly connecting a moveable, e.g. rotatable, part of the housing with the sound inlet, a position of the sound inlet may vary. By fixedly connecting a static part of the housing with the sound outlet, the position of the sound outlet may remain fixed, e.g. with respect to an ear canal. In combination, a distance between the sound inlet and sound outlet through the sound channel may thus be varied by moving the respective parts with respect to each other. For example, a length of the resonance cavity, formed in line with the sound channel may be varied for changing a frequency of the acoustic resonance. Alternatively, or additionally, both the sound inlet and sound outlet may be fixes with respect to a static part of the housing, and a moveable part may be used to change a size of a resonance cavity forming a side branch of the sound channel. By forming the sound outlet by an outlet part protruding from the housing, this part may be easily connected to any type of ear plug.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A illustrates a perspective view of a first acoustic device;
FIG 1B illustrates parts of the first acoustic device inside its housing;
FIGs 1C and 1D illustrate respective views of an adjustment mechanism of the first acoustic device arranged according to different settings;
FIG 2A illustrates a transparent perspective view of a second acoustic device;
FIGs 2B and 2C illustrate respective views of an adjustment mechanism of the second acoustic device arranged according to different settings;
FIG 3A illustrates a perspective view of a third acoustic device;
FIG 3B illustrates an exploded view of the third acoustic device;
FIGs 3C and 3D illustrate cross-section views of the third acoustic device;
FIG 4 illustrates a perspective view of a fourth acoustic device, with various cross-section view at the indicated positions;
FIG 5A illustrates a transparent perspective view of a fifth acoustic device, with cross-section views at the indicated positions;
FIG 5B illustrates separated parts of the fifth acoustic device;
FIG 6A and 6B illustrate graphs of frequency transmission spectra through model acoustic devices with different resonance characteristics.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

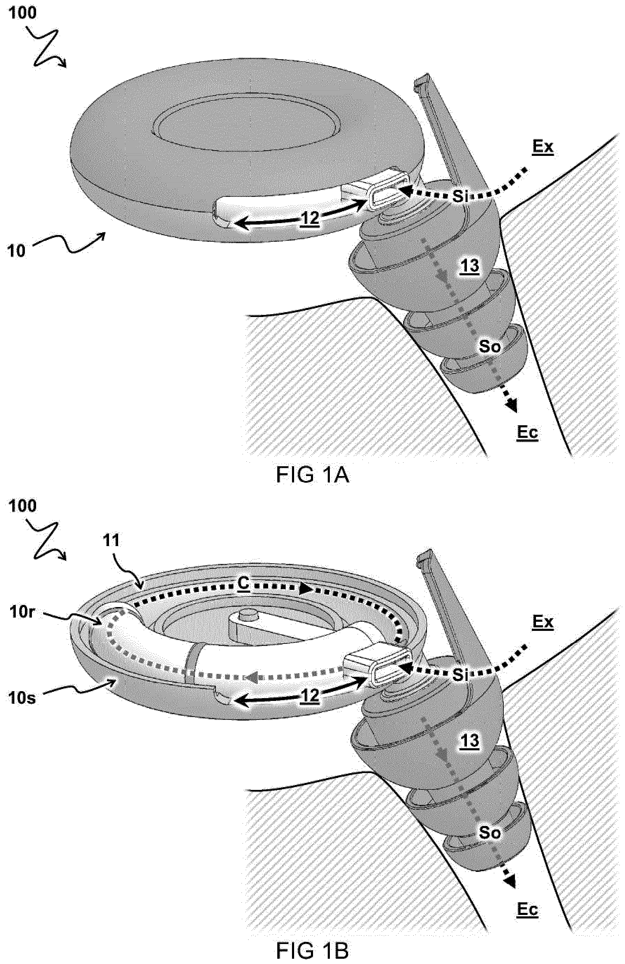

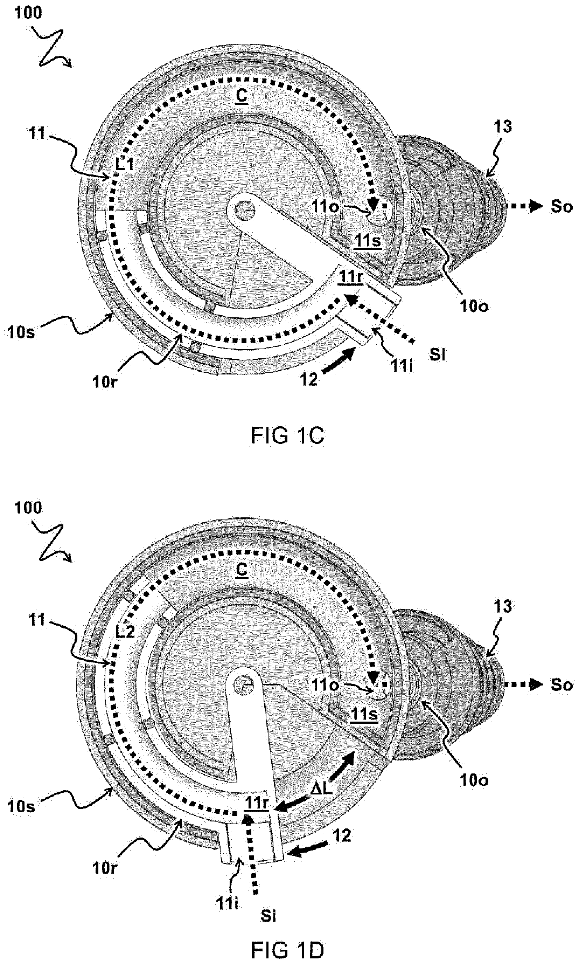

FIGs 1-5 illustrate embodiments of an acoustic device 100. As illustrated, the acoustic device 100 comprises a housing 10. A sound channel 11 extends through the housing 10 between a sound inlet 11i and a sound outlet 11o. The sound inlet 11i is configured to receive sound input "Si" from an external environment "Ex". The sound outlet 11o is configured to deliver sound output "So" that has passed through the sound channel 11 to an ear canal "Ec". A resonance cavity "C" is enclosed within the housing 10 and in acoustic communication with the sound channel 11. An adjustment mechanism 12 is configured to adjust a size of the resonance cavity "C".

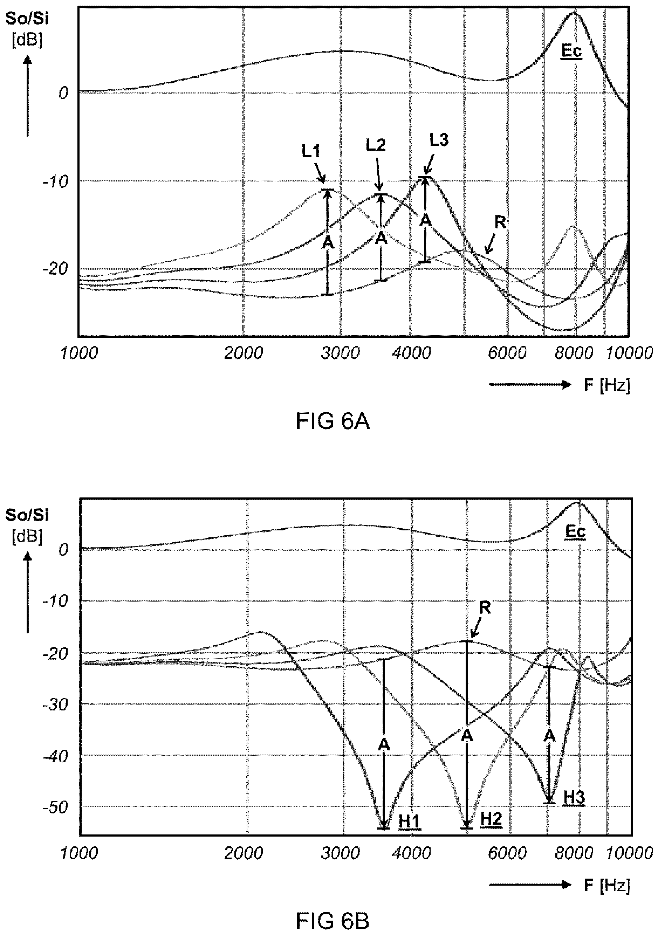

FIG 6A and 6B illustrate graphs of frequency transmission spectra through model acoustic devices with different resonance characteristics. In some embodiments, the resonance cavity "C" enclosed within the housing 10 and in acoustic communication with the sound channel 11 is configured to cause a respective acoustic resonance "A" in a frequency transmission spectrum So/Si of sound passing through the sound channel 11. In other or further embodiments, the adjustment mechanism 12 is configured to adjust a size of the resonance cavity "C" for controlling a frequency "F" of the acoustic resonance "A" in the frequency transmission spectrum So/Si. For example, the resonance cavity "C" has one or more adjustable dimensions and/or an adjustable volume resulting in a tunable acoustic resonance "A".

In some embodiments, e.g. as illustrated in FIG 6A, the resonance cavity "C" is configured to cause a tunable acoustic resonance formed as a peak on the frequency transmission spectrum So/Si. For example, this may be used to enhance speech or other sound of interest. To have a desired effect, the peak is preferably at least 5 dB, more preferably at least 10 dB, or more, above a reference background R, e.g. compared to the same configuration without the resonance cavity "C" and/or compared to a level where the peak is maximally tuned away to another frequency. In other or further embodiments, e.g. as illustrated in FIG 6B, the resonance cavity "C" is configured to cause a tunable acoustic resonance formed as resonance dip, also referred to as an anti-resonance, on the frequency transmission spectrum So/Si. For example, this may be used to attenuate and/or filter unwanted sound. To have a desired effect, the dip is preferably at least 10 dB, more preferably at least 20 dB, most preferably at least 30 dB, below a reference background R, e.g. compared to the same configuration without the resonance cavity "C" and/or compared to a level where the peak is maximally tuned away to another frequency. Also combinations of one or more peaks and one or more dips are possible, e.g. using one or more (tunable) resonance cavities.

In some embodiments, the acoustic resonance "A" is tunable to be within in a range of the human audible spectrum and/or in a range of frequencies associated with human speech. In other or further embodiments, a (central) frequency "F" of the acoustic resonance "A" is tunable in to be within a range between 20 Hz and 20,000 Hz (20 kHz), preferably within a range between 80 Hz and 5 kHz. Most preferably the resonance cavity "C" is tunable to cause the acoustic resonance "A" in a frequency range below 4 kHz. For reference, FIG 6A illustrates a series of resonance peaks caused by different length of a resonance cavity "C" in line with the sound channel 11. For example, the peak above 4 kHz was generated using a canal length L3=20mm; the peak between 3 kHz and 4 kHz was generated using a canal length L2=25mm; and the peak below 3 kHz was generated using a canal length L1=25mm. Similarly, FIG 6B illustrates peaks at different frequencies causes by respective (Helmholtz) cavities having different depths with H1 being the largest depth, and H3 being the shallowest depth. In general, it will be understood that, to achieve a relatively low resonance frequency, a respective dimension and/or volume of the resonance cavity "C" may be relatively large. Advantageously, the various solutions as described herein, may alleviate excessive dimensioning of the acoustic device 100, e.g. by "curling up" a dimension, such as the length, of the resonance cavity "C", in particular by having the length of the resonance cavity "C" arranged along a circular path.

In some embodiments, e.g. as shown in the embodiments of any of FIGs 1 -5, the resonance cavity "C" is arranged in line and/or in series with the sound channel 11. In other words the resonance cavity "C" may form part of a shortest acoustic path between the sound inlet 11i and the sound outlet 11o. For example, a resonance cavity in line with the sound channel 11 may be used to provide an adjustable resonance peak in the frequency transmission spectrum So/Si, e.g. as illustrated in FIG 6A. In other or further embodiments (not shown), the resonance cavity "C" is arranged in a side branch of the sound channel 11. For example, this may be a side branch of the shortest acoustic path between the sound inlet 11i and the sound outlet 11o. Such a side branch volume may act as a Helmholtz resonator. For example, as illustrated in FIG 6B, an (anti) resonance cavity in a side branch of the sound channel 11 may be used to provide an adjustable dip or anti-resonance in the frequency transmission spectrum.

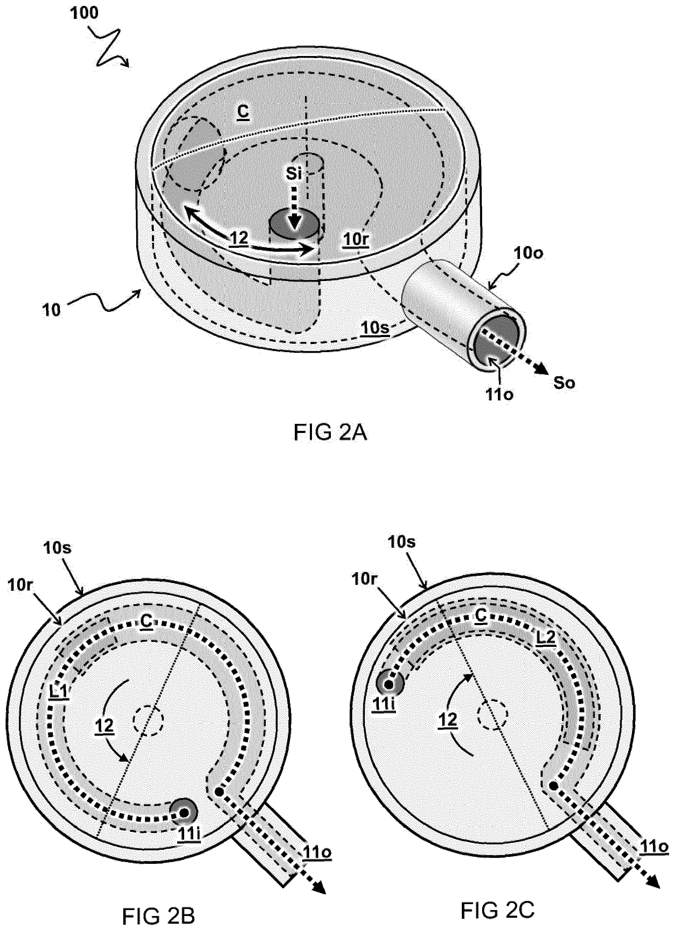

In a preferred embodiment, e.g. as illustrated in FIGs 1C-1D or 2B-2C, the adjustment mechanism 12 is configured to adjust the size L1,L2 of the resonance cavity "C" in a continuous range of values ΔL between a maximum size L1 and minimum size L2. Accordingly, the size of the resonance cavity "C" may be set to any value in the continuous range. This may allow precise tuning of the frequency transmission spectrum So/Si. For example, the size of the resonance cavity "C" may be tuned in accordance with a specific ear shape, e.g. ear canal size, and/or preference of the user. Most preferably, the adjustable size L1,L2 of the resonance cavity "C" comprises, or is essentially determined by, an adjustable length of the resonance cavity "C". For example, a characteristic length of the resonance cavity "C" may be determined by the longest dimension along an acoustic path of the sound passing through the sound channel 11, e.g. between a path between respective channel blocks 11r, 11s and/or other confining structures such as a relatively narrow opening, and/or an acoustic filter element such as a mesh and/or membrane.

In a preferred embodiment, the adjustment mechanism 12 is externally accessible from outside the housing 10 for manually adjusting the size L1,L2 of the resonance cavity "C". In some embodiments, the housing 10 comprises different parts, e.g. a first part 10r and a second part 10s, which are moveable with respect to each other. For example, said movement may change the size L1,L2 of the resonance cavity "C". In a preferred embodiment, the first part 10r is rotatably connected with respect to the second part 10s. For example, the adjustment mechanism 12 is configured to effect rotation of the first part 10r with respect to the second part 10s. Most preferably, said rotation may be used to change the size L1,L2 of the resonance cavity "C". In one embodiment, the first part 10r of the housing is fixedly connected the sound inlet 11i. In another or further embodiment, the second part 10s is fixedly connected to the sound outlet 11o.

In use, the sound outlet 11o (and thus the second part 10s) may be fixed with respect to the ear canal "Ec" while the first part 10r may be rotatable. In this regard, the first part 10r may be considered a rotatable part, and the second part 10s may be considered a static or fixed part. For example, the sound outlet 11o is formed by an outlet part 10o protruding from the housing for connection with an ear plug 13, e.g. formed of a resilient material fitting in an ear canal. While FIGs 1A-1D illustrate a universal ear plug, it will be understood that also a custom ear plug may be used, e.g. customized to fit a specific ear canal.

In a preferred embodiment, the first part 10r is configured to rotate with respect to the second part 10s along a circular trajectory. For example, the circular trajectory coincides with a part of the sound channel 11 forming the resonance cavity "C". More preferably, the resonance cavity "C" is formed by a tubular chamber extending along a circular trajectory. Most preferably, a length of the tubular chamber, e.g. along the circular trajectory, is adjustable by the adjustment mechanism 12 for controlling the frequency "F" of the acoustic resonance A in the frequency transmission spectrum So/Si. For example, the tubular chamber may be shaped like part of a donut. In some embodiments, the resonance cavity "C" is bounded at a first end of the circular trajectory by a first channel block 11r, and bounded at a second end of the circular trajectory by a second channel block 11s. For example, the first channel block 11r is configured to rotate inside the sound channel 11 along the circular trajectory when the first part 10r of the housing 10 is rotated with respect to the second part 10s of the housing 10. In another or further embodiment, the second channel block 11s remains static inside the sound channel 11 when the first part 10r of the housing 10 is rotated with respect to the second part 10s of the housing 10.

In one embodiment, the first channel block 11r is arranged proximate, e.g. adjacent, to the sound inlet 11i. In another or further embodiment, the second channel block 11s is arranged proximate, e.g. adjacent, to the sound outlet 11o. For example, the first channel block 11r is arranged within one centimeter from an entrance of the sound inlet 11i into the resonance cavity "C", preferably within half a centimeter, more preferably within 0.2 cm, within 0.1 cm, or flush with the entrance of the sound inlet 11i. For example, the second channel block 11s is arranged within one centimeter from an exit out of the resonance cavity "C" to the sound outlet 11o, preferably within half a centimeter, more preferably within 0.2 cm, within 0.1 cm, or flush with the entrance of the sound inlet 11i.

In some embodiments, the housing 10 comprises a sound filtering element, such as a mesh and/or membrane, disposed between the sound inlet 11i and the resonance cavity "C", and/or between the resonance cavity "C" and the sound outlet 11o. For example, the sound filtering element is disposed at an entrance of the sound inlet 11i into the resonance cavity "C", and/or at an exit of resonance cavity "C" to the sound outlet 11o. For example, such sound filtering element may be used to more effectively bound a respective volume in the resonance cavity "C".

Aspects of the present disclosure can also be embodied as method of using an acoustic device 100. Some embodiments, comprise receiving sound from an external environment "Ex" via a sound inlet 11i of the acoustic device 100, and passing the sound through a sound channel 11 extending through a housing 10 of the acoustic device 100 to deliver the sound that has passed through the sound channel 11 via a sound outlet 11o of the acoustic device 100 into an ear canal "Ec". Other or further embodiments comprise causing, by a resonance cavity "C" enclosed within the housing 10 and in acoustic communication with the sound channel 11, an acoustic resonance "A" in a frequency transmission spectrum So/Si of the sound passing through the sound channel 11. Advantageously, an adjustment mechanism 12 of the acoustic device 100 may be used to adjust a size L1,L2 of the resonance cavity "C" to thereby control a frequency "F" of the acoustic resonance "A" in the frequency transmission spectrum So/Si.

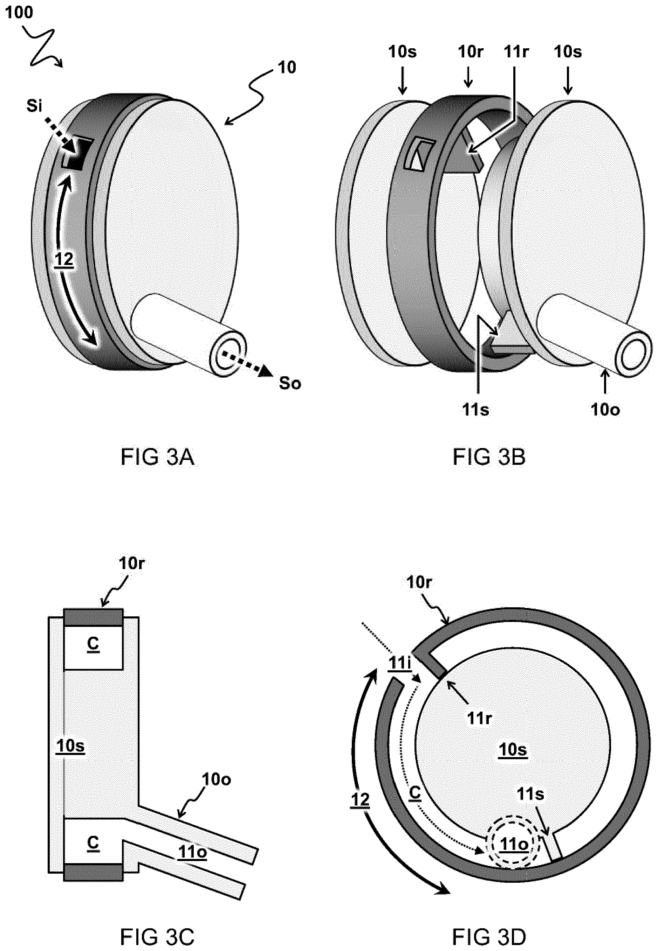

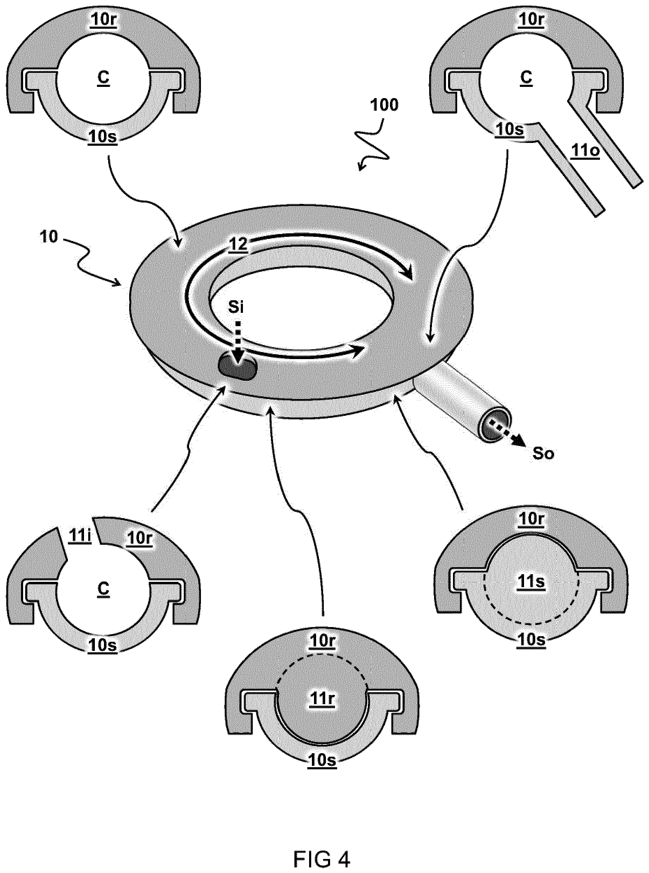

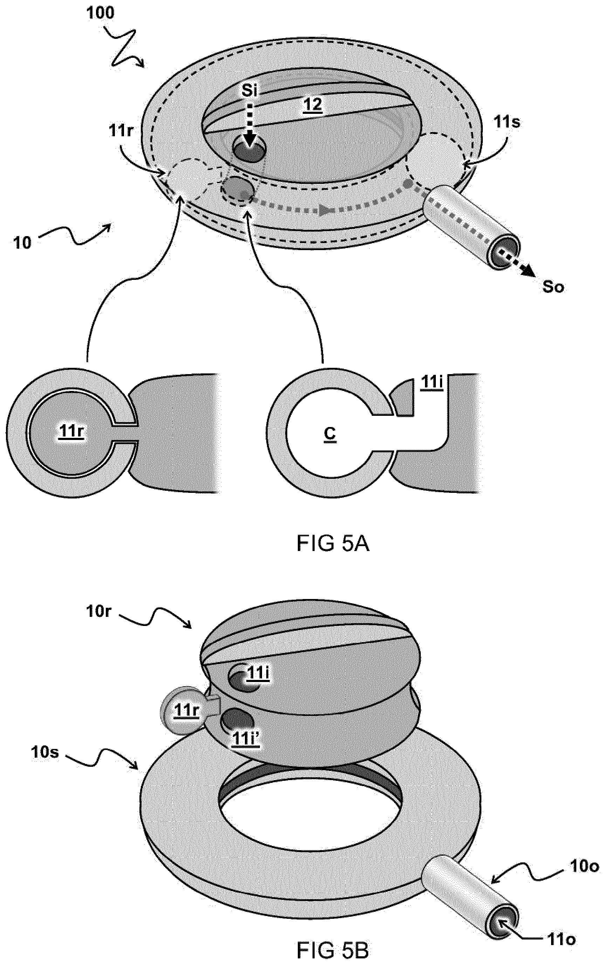

In some embodiments, e.g. as shown in FIG 1 and 3, the adjustment mechanism 12 comprises a knob or slider for adjusting the size L1,L2 of the resonance cavity "C". In other or further embodiments (not shown), a respective indication of a respective sound setting is printed on the housing 10 and/or adjustment mechanism 12. For example, scale of different sound settings may be printed one or both of the housing 10 and/or adjustment mechanism 12. In one embodiment, e.g. as shown in FIGs 1A - 1D, the sound inlet 11i forms a knob of the adjustment mechanism 12. In another or further embodiment, e.g. as shown in FIGs 3A - 3D, the sound inlet 11i is formed on a rotatable slider. In other or further embodiments, e.g. as shown, in any of FIGs 2 - 5, the adjustment mechanism 12 comprises and/or or couples to a rotatable part 10r of the housing 10. In one embodiment, e.g. as shown in FIGs 2A - 2C or FIGS 5A - 5B, the sound inlet 11i is formed on an inner rotatable part 10r of the housing, which is rotatable with respect to an outer static part 10s of the housing. In another or further embodiment, e.g. as shown in FIG 4, the rotatable part 10r and the static part 10s may be formed as upper and lower halves of a donut shaped housing. So, it will be appreciated that many variations can be envisaged without departing from the scope of the present disclosure.

For the purpose of clarity and a concise description, features are described herein as part of the same or separate embodiments, however, it will be appreciated that the scope of the invention may include embodiments having combinations of all or some of the features described. For example, while embodiments are shown in the figures for a resonance cavity "C" in line with the sound channel 11, also variations of this may be envisaged by those skilled in the art having the benefit of the present disclosure for achieving a similar function and result. For example, the resonance cavity "C" as illustrated in any of FIGs 1-5 may be converted to a side branch / Helmholtz resonator, depending on a placement of the sound inlet 11i and the sound outlet 11o, with respect to the resonance cavity "C". For example, the sound inlet 11i as shown in FIGs 1C and 1D, could be closed, and an alternative sound inlet (not shown) could be placed on a static part 10s of the housing 10, while the adjustment mechanism 12 may move the rotatable channel block 11r to determines a size of the side-branching resonance cavity "C". Similar adjustments can also be made to any of the other embodiments.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An acoustic device (100) comprising
a housing (10);
a sound channel (11) extending through the housing (10) between
a sound inlet (11i) configured to receive sound input (Si) from an external environment (Ex), and
a sound outlet (11o) configured to deliver sound output (So) that has passed through the sound channel (11) to an ear canal (Ec);
a resonance cavity (C) enclosed within the housing (10) and in acoustic communication with the sound channel (11) for causing an acoustic resonance (A) in a frequency transmission spectrum (So/Si) of sound passing through the sound channel (11); and
an adjustment mechanism (12) configured to adjust a size (L1,L2) of the resonance cavity (C) for controlling a frequency (F) of the acoustic resonance (A) in the frequency transmission spectrum (So/Si).

2. The acoustic device (100) according to the preceding claim, wherein the resonance cavity (C) is arranged in line with the sound channel (11), forming part of a shortest acoustic path between the sound inlet (11i) and the sound outlet (11o).

3. The acoustic device (100) according to any of the preceding claims, wherein the adjustment mechanism (12) is configured to adjust the size (L1,L2) of the resonance cavity (C) in a continuous range of values (ΔL) between a maximum size (L1) and minimum size (L2).

4. The acoustic device (100) according to any of the preceding claims, wherein the adjustable size (L1,L2) of the resonance cavity (C) is determined by an adjustable length of the resonance cavity (C).

5. The acoustic device (100) according to any of the preceding claims, wherein the resonance cavity (C) is formed by a tubular chamber extending along a circular trajectory, wherein a length of the tubular chamber along the circular trajectory is adjustable by the adjustment mechanism (12) for controlling the frequency (F) of the acoustic resonance (A) in the frequency transmission spectrum (So/Si).

6. The acoustic device (100) according to any of the preceding claims, wherein the adjustment mechanism (12) comprises and/or or couples to a rotatable part of the acoustic device (100).

7. The acoustic device (100) according to any of the preceding claims,
wherein the housing (10) comprises a first part (10r) and a second part (10s),
wherein the first part (10r) is rotatably connected with respect to the second part (10s);
wherein the adjustment mechanism (12) is configured to effect rotation of the first part (10r) with respect to the second part (10s);
wherein the rotation changes the size (L1,L2) of the resonance cavity (C).

8. The acoustic device (100) according to the preceding claim, wherein the first part (10r) is configured to rotate with respect to the second part (10s) along a circular trajectory, wherein the circular trajectory coincides with a part of the sound channel (11) forming the resonance cavity (C).

9. The acoustic device (100) according to any of the two preceding claims, wherein the first part (10r) of the housing is fixedly connected the sound inlet (11i) and the second part (10s) is fixedly connected to the sound outlet (11o).

10. The acoustic device (100) according to any of the three preceding claims,
wherein the resonance cavity (C) is bounded at a first end of the circular trajectory by a first channel block (1 lr), and bounded at a second end of the circular trajectory by a second channel block (11s);
wherein the first channel block (1 lr) is configured to rotate inside the sound channel (11) along the circular trajectory when the first part (10r) of the housing (10) is rotated with respect to the second part (10s) of the housing (10);
wherein the second channel block (1 ls) remains static inside the sound channel (11) when the first part (10r) of the housing (10) is rotated with respect to the second part (10s) of the housing (10).

11. The acoustic device (100) according to the preceding claim, wherein the first channel block (1 lr) is arranged adjacent to the sound inlet (11i); and the second channel block (1 ls) is arranged adjacent to the sound outlet (11o).

12. The acoustic device (100) according to any of the preceding claims, wherein the adjustment mechanism (12) is externally accessible from outside the housing (10) for manually adjusting the size (L1,L2) of the resonance cavity (C).

13. The acoustic device (100) according to any of the preceding claims, wherein the adjustment mechanism (12) comprises a knob or slider for adjusting the size (L1,L2) of the resonance cavity (C).

14. The acoustic device (100) according to any of the preceding claims, wherein the sound outlet (11o) is formed by an outlet part (10o) protruding from the housing for connection with an ear plug (13).

15. Method of using an acoustic device (100), the method comprising
receiving sound from an external environment (Ex) via a sound inlet (11i) of the acoustic device (100), and passing the sound through a sound channel (11) extending through a housing (10) of the acoustic device (100) to deliver the sound that has passed through the sound channel (11) via a sound outlet (11o) of the acoustic device (100) into an ear canal (Ec);
causing, by a resonance cavity (C) enclosed within the housing (10) and in acoustic communication with the sound channel (11), an acoustic resonance (A) in a frequency transmission spectrum (So/Si) of the sound passing through the sound channel (11); and
using an adjustment mechanism (12) of the acoustic device (100) to adjust a size (L1,L2) of the resonance cavity (C) to thereby control a frequency (F) of the acoustic resonance (A) in the frequency transmission spectrum (So/Si).
